(19)

**Europäisches Patentamt**
**European Patent Office**
**Office européen des brevets**

(11) **EP 4 066 933 A1**

(12) **EUROPEAN PATENT APPLICATION**
published in accordance with Art. 153(4) EPC

(43) Date of publication:
**05.10.2022 Bulletin 2022/40**

(51) International Patent Classification (IPC):
*B01J 13/20* (2006.01)     *B82Y 5/00* (2011.01)
*A01N 25/08* (2006.01)     *A01N 25/28* (2006.01)
*A01N 53/12* (2006.01)     *A01P 7/04* (2006.01)

(21) Application number: 20878307.6

(22) Date of filing: 23.10.2020

(86) International application number:
**PCT/ES2020/000051**

(87) International publication number:
**WO 2021/079017 (29.04.2021 Gazette 2021/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.10.2019 ES 201900166**

(71) Applicants:
• **Fundacion Centro de Innovacion y
  Demostracion Tecnologica
  28046 Madrid (ES)**
• **Qualitas Obras Servicios e Ingenieria S.L.
  46980 Paterna, Valencia (ES)**
• **Laurentia Technologies S.L.
  46980 Paterna, Valencia (ES)**
• **Universitat Politècnica de València
  46022 Valencia (ES)**

(72) Inventors:
• **PORCAR VIVES, Javier
  46004 Valencia (ES)**

• **CORVINOS AGUARÓN, Mᵃ Dolores
  46004 Valencia (ES)**
• **VILA GOMEZ, José Manuel
  46980 Paterna, Valencia (ES)**
• **DEVESA TARÍN, José
  46980 Paterna, Valencia (ES)**
• **LÓPEZ TENDERO, María José
  48980 Paterna Valencia (ES)**
• **LIMONES HERRERO, Daniel
  48980 Paterna Valencia (ES)**
• **PRADAS LÓPEZ, Sarai
  48980 Paterna Valencia (ES)**
• **CUENCA BUSTOS, Alejandro
  48980 Paterna Valencia (ES)**
• **GARCÍA GARRIDO, Eva María
  46022 Valencia (ES)**
• **BERNARDOS BAU, Andrea
  46022 Valencia (ES)**
• **MARTINEZ MAÑEZ, Ramón
  46022 Valencia (ES)**
• **NAVARRO LLOPIS, Vicente
  46022 Valencia (ES)**

(74) Representative: **Mora Granell, José Agustín
  G.V. Marqués del Turia 49, 6º, 3ª
  46005 Valencia (ES)**

(54) **SYSTEM FOR THE ENCAPSULATION AND CONTROLLED RELEASE OF PYRETHROIDS HAVING EXTENDED EFFICACY OVER TIME AGAINST FRUIT FLY**

(57)     System for the encapsulation and controlled release of pyrethroids having extended efficacy over time against fruit fly.

The present invention relates to a system for the encapsulation and controlled release of insecticides to combat Diptera, known as fruit fly, including the families Tephritidae and Drosophilidae. The system protects insecticide molecules against the processes of photolysis and only activates the release of the molecules by direct contact with the flies. The invention is based on the use of mesoporous silica particles as an encapsulating system, and on the functionalisation of the particles with protein molecules. The proteins have a dual function: on the one hand, they are substances that attract the flies; and on the other, they close the pores and only allow them to open when the fly, by contact, releases proteases and other fluids that degrade the protein covering. The formulations with pyrethroids encapsulated in these protein-functionalised mesoporous silica systems retain their insecticide activity after 35 days.

**Description**

The invention entails a system for the encapsulation and controlled release of pyrethroids to fight against fruit flies that prolongs over time.

**DESCRIPTION OF THE ART**

The present invention is related to the field of embedded and repellent compounds of pyrethroid nature, more specifically to systems that administer such compounds based on mesoporous silica particles, whose effectiveness prolongs over time once applied.

**BACKGROUND OF THE INVENTION**

Fruit flies are one of the most harmful pests to affect Mediterranean agriculture. Although this pest is endemic to the Mediterranean Region, it affects fruit crops worldwide. It affects not only the sweet fruit that mature at the end of spring, but also early and late citrus varieties. In recent years, its incidence has been further aggravated by extending areas where very early citrus varieties are grown, which are extremely sensitive to attacks by this pest when they fully ripen early in autumn when temperatures are still high. In 2011, the European and Mediterranean Plant Protection Organization indicated in an article that "Ceratitis capitata is the most serious pest for citrus and many other fruit in the majority of countries with a warm, Mediterranean tropical or subtropical climate" [EPPO Bulletin [0250-8052] 2011 vol: 41 iss: 3 p: 340 *Ceratitis capitata*]. The economic losses that this pest causes are vast because rotten fruit is completely unfeasible for commercial purposes, and causes problems for warehouses, transport and distribution. Besides, as *Ceratitis* is regarded as a quarantine pest in some third-party countries, the presence of eggs or larvae in fruit can lead to some lots delivered there being rejected and, thus, severe restrictions are imposed on Spanish exports to them [Primo Millo, Eduardo. Agriculture [00-1334] Año: 2004 Iss: 867 pág.: 790-703. Plan Valenciano de Actuacion Contra la Mosca de la Fruta (Ceratitis capitata Weidemann) campaña 2004]. The trend followed to control this pest has been to use increasingly more active substances or to substitute them for more active ones. The indiscriminate use of these active substances has led to environmental problems, and also to resistance to the active principles themselves (Couso Ferrer, F. Arouri Rabah; Beroiz, B; Perera, N; Cervera, A; Navarro-Llopis, V; Catañera, P; Hernandez-Crespo, C; Ortega F (2011). Cross-Resistance to insecticides in a Malathion-Resistant Strain of Ceratitis Capitata (diptera: Tephritidae). Journal of Economic Entomology. 104 (4): 1349-1358. Doi: 10. 1603/EC11082). This has meant having to regulate its use by prioritising prevention over eradication. Poor pest biodegradability, its high bioaccumulation power in the food chain, its toxicity in air, soil and water, and human intoxication by either skin contact or intake, have led to strict norms being imposed about its use in many countries. Notwithstanding, these biocides still need to be employed. This entails searching for new molecules with more environmentally friendly properties, or developing processes or techniques that allow the impact of these biocides to be targeted on pests to, thus, minimise side effects on the environment. So, it is not surprising that one of the main challenges is to search for alternative methods associated with the biological control of certain populations, but without interfering with the natural balance of the involved species. New population control techniques particularly seek treatment specificity, which means that correctly differentiating the insect species from others is fundamental.

Fruit flies are one of the most difficult insects to combat due to:

- The ease with which they adapt to the environment. When this fly finds a suitable habit with no competition by other individuals of the same species, it colonises it in very short time periods.

- Its high reproduction potential:( it lays from 75 to 150 eggs between 5 and 6 times during its lifetime, about 1 month.)

- its ability to fly, which enables it to find a safe way to escape from treated zones.

- its rapidly developing resistance to pesticides.

- the reduction in the number of authorised active materials.

- no suitable biocidal formulation is available to combat it.

Therefore, a new technique called integral pest management (IPM) has been developed. It combines several control methods: physical controls by means of barriers, traps, etc.; biological controls using pathogens, microorganisms, fungi, nematodes, parasitic insects, among others, which act as predators of the pest's causative agent; finally, chemical controls (Romero F, Manejo Integrado de Plagas. Las bases, los conceptos y su mercantilizacion, 2004, Universidad Autónoma de Chapingo, Mexico, pp. 1-37).

Pyrethrins are one of the safest existing insecticides because they attack the insect nervous system, but do not affect other animals and plants (Wandhwa P, Ranst EV. Damme PV, Pyrethrum (Chrysanhemum cinerariaefolium Vis.) Cultivation in West Kenya: origin, ecology conditions and management. Industrial Crops and Products, 1996; 5: 307-322). However, pyrethrins are easily degraded in the presence of light and, thus, their efficiency is very quickly lost after being applied. Pyrethroids are synthetic organic compounds that derive from the aforementioned pyrethrins (Hill IR. Leahey J p. The pyrethroid insecticides, 1985, Taylor & Francis, London (UK), pp. 151-247). As they are not very toxic for mammals and humans, pyrethroids have been well-accepted and are widely applied worldwide. However, they are toxic for some organisms, such as bees, fish and other aquatic organisms. Currently, the pyrethroid insecticides that are chemically classified as esters are the most widespread because they exhibit good insecticidal properties for a wide range of applications. As they are still quite unstable to air and light, their use in agriculture implies some limitations.

Introducing microencapsulation processes allows safer products to be developed for both workers and the environment. Encapsulation can be carried out in the form of molecules, solid particles, or liquid beads to give way to micron-sized particles. The product that results from this technological process is known as "microparticles" (capsular system), "microcapsules" (matrix system) or "microspheres" (indistinctively by being only differentiated by their spherical shape). These systems differ depending on their morphology and internal structure, but they all share a common characteristic: their particle size, which is always < 1 mm. When particle size is < 1 $\mu$m, the product that results from the microencapsulation process is called "nanospheres", "nanoparticles" or "nanocapsules".

The variety of materials that can be employed for microencapsulation is gradually increasing as new biomaterials arise and new applications are profiled. Among them we find: active substances microencapsulated with a water soluble or water dispersible, comb shaped polymer. (ES 2 237 668 T3. European Patent, 2005).

They include the following:

- Encapsulants: inorganic matter (silica, silicates), lipids (waxes, paraffins, oils or fats in the form of liposomes), proteins (i.e. gluten, casein, albumin, soya, caseinates, gelatine), gums (sodium alginate, gum arabic), carbohydrates (starch, dextrins, cellulose, chitosan) and polymers

- Solvents: ethyl acetate, acetone, dichloromethane, chloroform, methanol, propylene glycol or acetonitrile, which properly dissolve the employed active substances and are relatively miscible with water

- Emulsifiers: Hydrophobic polymeric colloids, non-ionic surfactants, polyvinyl alcohol or similar

- Preservatives: the usual ones include sodium benzoates or sodium nitrites, among others, like Prevenol® and Proxel®

- Attractants, depending on the pest to be treated

Others: synergistic ones for increasing biocidal function, antifoam ones that derive from silanes like poly(dimethyl siloxane) and magnesium stearate, thickeners (xanthan or silicic acids), buffers (all weak acids and their salts, but preferably phosphate buffer, carbonate buffer and citrate buffer), cold stabilisers (including urea, glycerol and propylene glycol), neutralising agents, deodorisers and colour polymers

For active principle release, insecticide can be released from the microcapsule by two mechanisms:

- diffusing through the capsule wall or via a membrane covering the wall. Permeability through the matrix and the solubility of the capsule wall component influence the diffusion rate

- destroying the microcapsule wall by either physical processes (normal solution in water, shear stresses) or chemical reactions (changes in pH or osmotic pressure, hydrolysis, thermal or enzymatic degradation)

The encapsulation of such compounds has recently began to be studied in mesoporous silicon oxide particles as an alternative administration form.

In the literature, mesoporous silica particles, or MSP, are considered to be biocompatible materials; for example, the study by Wehling et al (A critical study: assessment of the effect of silica particles from 15 to 500 nm on bacterial viability". Environmental Pollution, 176, 292-299, 2013), investigated the effect of silica particle sizes between 15 and 500 nm on bacterial viability. The results showed that particles showed no inhibitory properties regardless of their particle size.

The load of biocidal compounds inside MSP pores has been extensively studied. For example, in the article of Liu et al ("Porous hollow silica nanoparticles as a controlled delivery system for water-soluble pesticide". Materials Research Bulletin 41 (2006) 2268-2275), a validamycin water-soluble insecticide was encapsulated, and a release profile was obtained in different phases because part of the insecticide was adsorbed on the surface. Another work by Chen et al (Slow-Release Formulation of a New Biological Pesticide, Pyoluteorin, With Mesoporous Silica. J Agric. Food Chem. 2011, 59 (1), pp. 307-311) developed a biological insecticide encapsulation system, Pyouteroyn, in mesoporous silica with a continuous release profile of 85% in 28 days.

In most cases, the aim is to not only control the release profile, but to also provide protection to unstable insecticides that easily degrade. Thus, for example, Wang et al (Construction and evaluation of a controlled release delivery system of Abamectin using porous silica nanoparticles as carriers". Nanometric Research Letters, December 2014, 9: 655) were able to improve instability against Abamectin light.

Patent WO2017091096A2 (2016) reveals a process of encapsulating biocidal essential oils in mesoporous silica systems using silicate precursors and siloxane. In this patent, the ultimate application is to release these essential oils as biostimulants of plants *Passiflora incarnata* L and *Momordica charantia L.*

European patent EP2662069A2 (2013) refers to mesoporous material in which at least some pores are loaded with essential oils. However, the encapsulation process ends by covering pores with a biodegradable polymer film. Over time, mesopore film cover degradation determines variation in the essential oil release rate with time.

Although the encapsulation of active compounds in mesoporous particles provides advantages for, for instance, the controlled and sustained release of a compound, these techniques are not entirely satisfactory. For example, a disadvantage with such a technique is that once active compounds have been released from mesoporous particles, they remain free in the environment, which implies the same drawbacks as individually applied compounds (e.g., solubility, volatility with less effectiveness against pests by low persistence, unpleasant taste, etc.). As the released compounds are dispersed in the environment, a substantial concentration of them cannot be obtained in a particular area of interest. Additionally, introducing these compounds into pores is not always possible because of polarity ratios and pore size/compound ratios. Using porous substances with large-diameter pores makes their closure and, therefore, the encapsulation of the added compound difficult.

Patent WO/2018/096098 discloses an antimicrobial essential oil encapsulation system based on saccharide-coated MSPs (e.g., starch, maltodextrin, maltose, glucose). This invention provides a release system induced by the exogenous enzymes of Aspergillus *niger* fungi. When these fungi are absent, antimicrobial oil release is very low. Conversely, the presence of these fungi activates release with very high levels of antimicrobial oils. This patent is an example of controlled release that maintains a substantial concentration of the active compound for a longer period. However, it was designed to specifically fight against *Aspergillus niger*.

The technique still needs an insecticide release system against fruit flies (tephritidae and drosophilidae) that leaves a substantial concentration of the insecticidal compound for a longer time period. The present patent demands a method that is able to maintain insecticides occluded in particles and are released only by direct contact with flies. This will be possible thanks to the functionalisation of the surface of the particles with protein molecules that close pores, and their opening only occurs when flies are in direct contact and, thus, releases proteases and other digestion substances to degrade the protective protein cover.

## DESCRIPTION OF THE INVENTION

This invention refers to a nanodevice based on an encapsulation and controlled release system of insecticides to fight against fruit flies. The nanodevice consists of MSP (i.e. MCM-41, SBA-15) in which the insecticide is encapsulated, and molecules are released only when they are in direct contact with flies. Apart from the nanodevice controlling insecticide release, it provides protection against photolysis processes.

The presented invention relates to a specific mesoporous material for fighting against insects. It is based on using molecules with insecticidal activity encapsulated in protein-functionalised mesoporous silica nanoparticles. This material allows controlled active ingredient release exclusively in the presence of a specific stimulus, such as insects. The released load is activated by the presence of proteases and by insects' digestive and excreted substances.

Obtaining MSP is well-defined in the state of the art by different sol-gel methods from siloxane precursors by several techniques in which surfactants are used as molecules that direct the mesoporous structure. MSP may contain pores from 0.5 to 10 nm, but preferably from 1.5 to 3.5 nm, with a particle diameter of 10 nm to 250 nm, 250 nm to 500 nm, 500 nm to 1 micron, but preferably from 25 nm to 150 nm. Mesoporous particles may have a specific BET surface of between 10 $m^2/g$ and 1,500 $m^2/g$, but preferably between 200 $m^2/g$ and 700 $m^2/g$

The insecticide can be placed inside the mesoporous structure of silica particles by impregnating these preformed particles. Alternatively, they can be introduced by the same particle formation process by following a sol-gel process in mini-emulsion. In both cases, the quantity of the insecticide to be released, and can be encapsulated, is 0.01 to 0.50 g per support g.

Different synthetic pyrethroids can be encapsulated against fruit flies, such as allethrin, phenvalerate, phyuvalinate, cyfluthrin, bifenthrin, fenpropatine, resveratrol, sumithrin permethrin, esfenvalerate, cimetidine, lambda cyhalothrin, deltametine, tefluthrin, tetraprine and tralomemine and/or any mixtures thereof.

The main innovation of this invention lies in chemical functionalisation with proteins on the surface of silica particles. Proteins perform a double function; on the one hand, they are attractants of flies. On the other hand, they close pores and only allow them to open when flies attempt to eat substances and release the proteases that degrade the protein shell. The formulations with pyrethroids or pyrethroids encapsulated in protein-functionalised mesoporous silica systems retain insecticidal activity after 35 days.

The silica particles obtained present hydroxyl groups on the surface of pores. These groups are capable of covalently bonding to the functional groups of the attractant protein. N-(3-Dimethylaminoethyl)-N,-ethylcarbodimide (EDC) is used to activate the carboxylic groups of the amino acids of proteins. The protein concentration in the particle suspension or emulsion can range by 5-30%, but preferably by 10-15%.

The present invention offers the following advantages:

- More specific and more controlled release.

- Release occurs selectively in the presence of fruit flies that excrete proteases.

- The excreted proteases lead to the hydrolysis of the protein cover of particles and, thus, pores remain open and the insecticide is released.

The first aspect of the presented invention refers to a nanodevice for the controlled release of substances. It comprises a protein-coated mesoporous support, and the aforementioned proteins are attractants for certain strains of insect families like tephritidae or drosophilidae.

Its second aspect refers to the procedure that the nanodevice follows, which comprises these steps:

a) synthesis of the silica mesoporous support,

b) suspension of the support in a solution of the substance to be released,

c) addition of excess protein to the above suspension.

Steps a) and b) can be optionally carried out simultaneously as one-step mini-emulsion processes.

Its third aspect refers to a composition that comprises nanodevices like those previously described.

Its fourth aspect refers to using the nanodevice as previously indicated to prepare an insecticide.

Its fifth aspect refers to using the nanodevice as previously indicated to prepare an insecticide to remove insects that excrete proteases.

## BRIEF DESCRIPTION OF DRAWINGS

In order to complement the provided description and help to better understand the features of this invention, it comes with a set of drawings that form an integral part of the description. This is an illustrative and non-limiting way to represent the following:

FIG. 1 Schematic of the release of insecticide from the protein-coated mesoporous silica material in the presence of fruit flies.

FIG. 2 The invention relates to a) an X-ray diffraction patterns of the post-synthesis (A) and calcined (B) silica mesoporous nanoparticles; b) the transmission electron microscopy (TEM) images of the nanodevices of this invention, silica mesoporous nanoparticles (A) and the loaded and functionalised solid (B); c) nitrogen adsorption-desorption of the calcined mesoporous material (a) and the final loaded and functionalised solid (b).

FIG. 3 Scanning electron microscopy image of the alternative porous silica particle support obtained by mini-emulsion.

FIG. 4 Detail of the application of formulations on leaves.

FIG. 5 Mortality as a % caused by clementine leaves after 1 h of exposure according to leaf ageing time.

FIG. 6 Mortality as a % caused by clementine leaves after 3 h of exposure according to leaf ageing time.

FIG. 7 Mortality as a % caused by clementine leaves after 24 h of exposure according to leaf ageing time.

FIG. 8 The attraction of the different formulations according to ageing time.

**PREFERRED EMBODIMENTS**

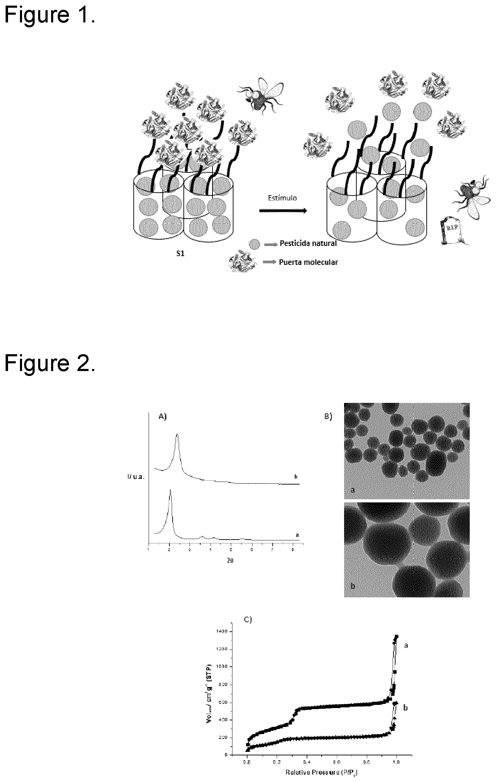

Below some examples are described to show the different studies that have led to the present invention, which refer to Figures 1 to 8.

Example 1. Silica mesoporous material synthesis.

The hexagonal MCM-41 phase will be used as nanoparticulate silica mesoporous material. Its synthesis methodology is based on the condensation of silica precuring (sodium silicate, tetraethylorthosilicate or tetramethylammonium silicate) in the presence of cationic surfactants under basic conditions. For the synthesis of the MCM-41-type inorganic nanoparticulate mesoporous support, tetraethylorthosilicate (TEOS) is employed as a hydrolytic inorganic precursor, with hexadecyltrimethylammonium bromide (CTABr) as a surfactant. Firstly, 1 g (2.74 mmol) of CTABr is weighed and dissolved in 480 ml of deionised water. Next 3.5 ml of 2M NaOH solution are added and the mixture's temperature is adjusted to 80°C with mild stirring. Next 5 ml of TEOS (25.7 mmol) are quickly added with strong stirring. This mixture is allowed to stir at 80°C for 2 h. The resulting powder is centrifuged and washed with water. The solid is dried at 70°C. To prepare the final porous material (MCM-41-type nanoparticles) and to remove the surfactant, the mesoporous solid is calcined following this temperature program me: temperature is increased from 25°C to 550°C within an interval lasting 3 h and 30 minutes, before being left at 550°C for 5 h and 30 minutes.

Example 2. Synthesis of the encapsulation and controlled insecticide release system for fighting against fruit flies (FIG. 1).

On a watch glass, 100 mg of ground MCM-41-type nanoparticles are homogeneously distributed. In a vial, 36 mg of lambda cyhalothrin are dissolved in 1 ml of $CH_2Cl2$ and weighed. This volume is poured dropwise onto the watch glass containing MCM-41 to run the encapsulation process by the impregnation method. The watch glass with the solid and pyrethroid solution is left overnight at 37°C in an oven to remove the solvent by evaporating it.

Having obtained the solid with the encapsulated pyrethroid (S1), in a 100-ml round-bottom flask 72.4 g of N-(3-Dimethylaminoethyl)-N ethylcarbodi (EDC) are weighed to activate the carboxylic groups of the amino acids of proteins with 50 g of corn protein hydrolysate. Both reagents are stirred in 37 l of distilled $H_2O$ for 30 minutes. After this time, 135.7 g of solid S1 are added. The suspension is stirred at room temperature overnight until all lumps have disappeared.

Example 3. Characterisation of the material. (FIG. 2).

In order to characterise the silica mesoporous nanomaterials herein synthesised, the following techniques are applied: powder X-ray Diffraction (PXRD), transmission electron microscopy (TEM), adsorption desorption of $N_2$.

FIG. 2A depicts the powder X-ray diffraction patterns (PXRD) of the synthesised MCM-41 and calcined MCM-41 solids. The low-angle PXRD of the synthesised MCM-41, prior to calcination (curve a), shows the four representative reflection planes of the hexagonal structure, which can be assigned to Bragg planes (100), (110), (200) and (210), with a cell parameter $a_0$ whose value is 43.83 Å (distance between planes d100 = 38 Å). In Figure 2, on the PXRD of the calcined MCM-41 sample (curve b), we can see a significant shift of the (100) reflection. This displacement is due to cell contraction of about 6-8 Å. The displacement and widening of peaks (110) and (200) are probably due to the condensation of silanols on the material's surface during the calcining process.

The mesoporous structure of the synthesized MCM-41 and calcined MCM-41 solids is also observed by TEM. The TEM images of the synthesised MCM-41 and the calcined MCM-41 solids (FIG. 2B) show the typical disordered hexagonal porosity of the MCM-41 matrix.

Another technique used to characterise the pore system of MCM-41 is to analyse the adsorption-desorption isotherms of $N_2$ (FIG. 2C). At the liquid nitrogen temperature (-196°C), this technique allows the surface area, size and pore volume of the material to be obtained. The $N_2$ adsorption-desorption isotherms of the calcined MCM-41 phase show a characteristic step of the MCM-41 mesopores at intermediate pressure values (0.2<P/PO<0.4), which corresponds to isotherm type IV. This step is due to N condensation by capillarity in mesopores. The fact that there is no hysteresis cycle within this interval and the curve's shape suggest the existence of uniform cylindrical mesopores with a pore diameter of 2.4 Nm and a pore volume of 0.47 $cm^3g^{-1}$, calculated by the Barret-Joyner-Halenda (BJH) method. The Brunauer-Emmett-Teller (BET) method gives a value of 368 $m^2g^{-1}$ for the specific total surface.

Example 4. Scaling the silica mesoporous material based on the CTAC surfactant.

The MCM-41-type inorganic mesoporous support is synthesised by replacing the CTABr surfactant with CTAC, hexadecyltrimethylammonium chloride. Firstly, 100 g of CTAC are dissolved in 48 L of distilled $H_2O$. Then 350 ml of 2M NaOH are added to the CTAC solution in water and heated to 80°C with gentle stirring (200 rpm). Next 500 ml of TEOS is added dropwise. Stirring is performed for 2 h at 600 rpm to obtain a white precipitate. This precipitate is centrifuged and placed in a mixture of ethanol and 37% HCl (ethanol: HCl ratio, 9 v/v), which is left at 80°C for 10 h to remove the surfactant. The solid is filtered and dried at 70°C for 18 h.

Example 5. Synthesis of the encapsulation and controlled insecticide release system for fighting against fruit flies **(ATAECINA formulation).**

In a stainless steel kneader, 1.66 g of the MCM-41 nanoparticles from Example 4 are homogeneously distributed. Then 0.6 g of lambda cyhalothrin is weighed and dissolved in 33.2 ml of $CH_2Cl_2$. This volume is poured dropwise into the kneader containing the MCM-41 nanoparticles to run the encapsulation process by the "kneading" method. The resulting solid is dried in a rotary extractor in a vacuum at 40°C. Having obtained the solid with the encapsulated pyrethroid, along with 614 g of distilled water, 1.2 of N-(3-dimethylaminoethyl)-N'-ethylcarbodimide (EDC) and 0.83 g of corn protein hydrolysate are placed inside a reactor. The mixture is stirred for 10 h.

Example 6. Scaling the silica mesoporous material based on surfactant CETRIMIDE.

The inorganic MCM-41-type mesoporous nanoparticulate support is synthesised using surfactant cetrimide, which is industrial-grade hexadecyltrimethylammonium chloride. Then 1 kg of cetrimide is dissolved in 480 l of distilled $H_2O$. Next 3.5 L of 2M NaOH are added to the cetrimide solution in water and are heated to 80°C with gentle stirring (200 rpm) and 5 l of TEOS are added dropwise. Stirring takes place for 2 h at 600 rpm and a white precipitate is formed. This precipitate is centrifuged and placed inside a mixture of ethanol and 37% HCl (ethanol: HCl ratio, 9: 1 v/v), which is left at 80°C for 10 h to remove the surfactant. The solid is filtered and dried at 70°C for 18 h.

Example 7. Synthesis of the encapsulation and controlled insecticide release system to fight against fruit flies **(Formulation Form 1).**

In a stainless steel kneader, 0.28 g of the mesoporous silica nanoparticles obtained in Example 6 are homogeneously distributed. Next 0.1 g of lambda cyhalothrin is weighed and dissolved in 2.78 ml of $CH_2Cl_2$. This volume is poured dropwise into the kneader containing the MCM-41 nanoparticles to run the encapsulation process by the "kneading" method. The resulting solid is dried in a rotary extractor in a vacuum at 40°C. Having obtained the solid with the encapsulated pyrethroid, along with 102.5 g of distilled water, 0.2 g of N-(-dimethylaminoethyl)-N'-ethylcarbodimide (EDC) and 0.14 g of corn protein hydrolysate are placed inside a reactor. The mixture is stirred for 10 h.

Example 8. The invention refers to a system to encapsulate the natural pyrethrum extract (natural insecticide) to fight against fruit flies that is obtained by mini-emulsion **(Formulation Form 2)**.

To a beaker the aqueous phase is added, which consists in 150 ml of distilled water and 6 ml of (3-aminopropyl) triethoxysilane (APTES). Stirring at 600 rpm is performed to allow the APTES hydrolysis and to obtain a homogeneous solution. In another beaker, the oil phase is mixed (8 g of natural pyrethrum extract and 18 ml of methyltrimethoxysilane (MTMS)) until a homogeneous solution is obtained. The oil phase is poured slowly over the aqueous phase and is stirred for 10 minutes. The resulting solution is centrifuged for 5 minutes at 5,000 rpm. The process is repeated by washing with distilled water twice to ensure that the solid is free of starting reagents. It is left to dry at room temperature.
Once the encapsulated pyrethrum oil has been obtained, 111 ml of distilled water are placed inside a beaker, and 0.15 g of corn protein and 0.22 g of EDC are added. After 30 minutes, 0.3 g of capsules are added and stirred firstly at 800 rpm. Once hydrophobicity is lost, stirring is done at 500 rpm for 10 h.

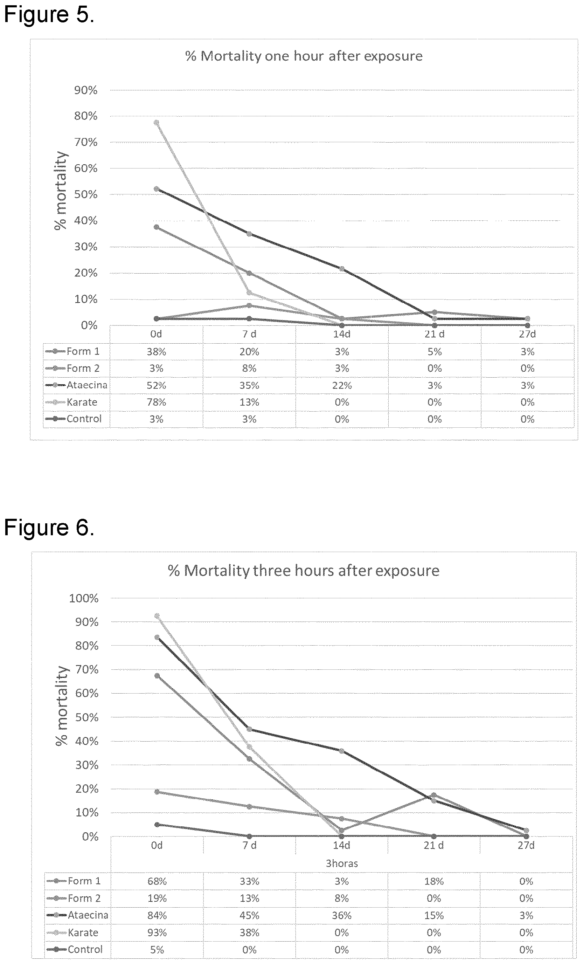

Example 9. Field Testing. (FIGS. 5, 6, 7 and 8).

At the CEQA IAM of the UPV, different formulations have been tested to study their insecticidal activity. Tests were performed under real field conditions during the fruit-ripening period, when fruit is more sensitive to Mediterranean fly attacks and fly populations are bigger. Tests were performed on mandarin leaves of a clemennules variety of cases.
For the leaf measurement tests of insecticidal activity, the following formulations were tested:

| Formula | Name |
|---|---|
| Example 5 | Ataecina |
| Example 7 | Form 1 |
| Example 8 | Form 2 |
| Water | Control |
| λ- Cihalotrin (0.12%)+Nulure(1.5%) | Karate |

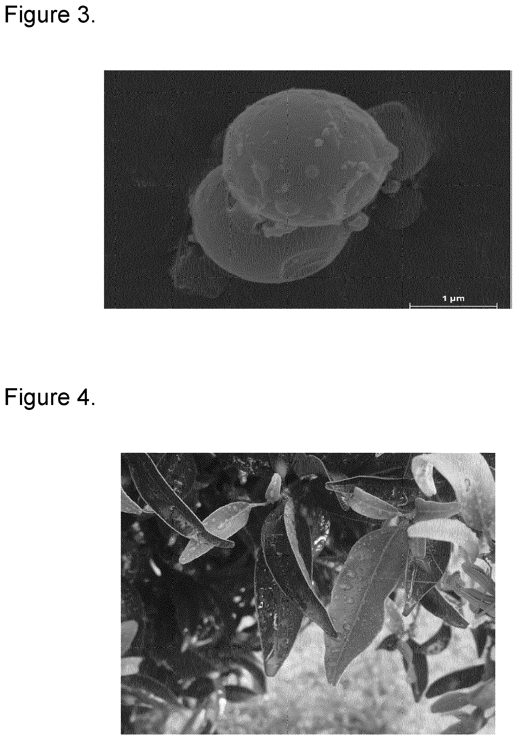

All the formulations are diluted to 5% in water, except for the Cyhalothrin formulation containing 1.5% Nulure (v: v) and 0.12% Lambda Cyhalothrin in the Karate Zeon formulation (Syngenta). Formulations are sprayed using a manual pressure sprayer (Matabi) on clementine leaves (FIG. 4). Treated leaves are labelled and allowed to age under real field conditions for 4 weeks. A sample of 18 leaves from each treatment is collected weekly and tested for insecticidal activity. To do so, these leaves are offered to flies by placing two leaves in each cage. The methacrylate cages (10 × 10 × 10 cm) contain five male and five female sexually mature *Ceratitis capitata* adults that had been fasted for 24 h to ensure eagerness to ingest bait. Leaves are left inside cages, where they remain for 1 h at flies' disposal. The flies that approach leaves are counted to study the attractant capacity of bait. Leaves are removed after 1 h and a maintenance diet (sugar, proteins, water) is left inside cages.

Attraction is studied during each test, and is measured as flies are attracted to the tested baits during a 1-hour period. Insecticidal activity is measured as mortality and the knock-down effect at 1 h, 3 h and 24 h after offering insects the treated baits.

Each ageing time and each formulation are tested every time in four cages. As FIG. 5 depicts, the formulation with Karate Zeon and proteins is that which more rapidly kills flies when freshly applied. However, this effect disappears 7 days after its application, when insecticidal activity is very poor. Ataecina is the only formulation to maintain the rapid insecticidal effect (only 1-hour exposure to leaves) for 14 days.

For mortality after 3 h of exposure (FIG. 6), once again Ataecina is the formulation with the most activity after 14 ageing days, and even exhibits good residual activity with leaves left in the field to age for 21 days. It should be noted that while the activity of formulation 1 is very poor after 14 ageing days, it apparently recovers at 21 days. Thus we can state that formulation 1 offers good ageing performance. As no explanation can be found for this reduction at 14 days, and despite recovery at 21 days, this value should be cautiously taken.

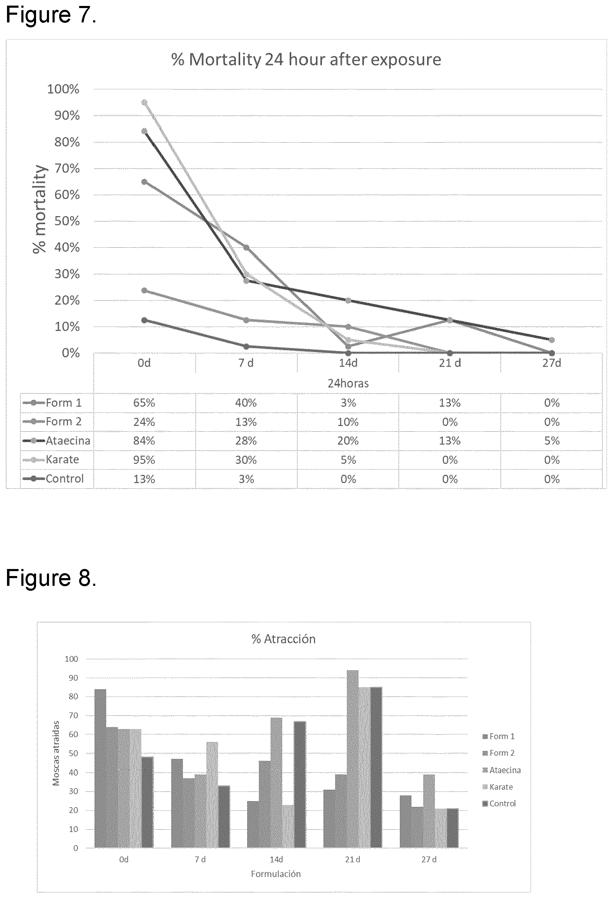

Finally, if we consider the mortality in FIG. 7, which is considered definitive (at 24 h of exposure), we find that Ataecina is the only formulation to remain active even 1 month after being applied.

When comparing the two alternative formulations, once again formulation 1 is more active than formulation 2. Formulation 1 is more active at 7 ageing days, and is even slightly better than the Ataecina formulation. The results on the attraction that different formulations obtain are shown in FIG. 8. These results reveal that, although formulation 1 presents good attraction when recently applied, this attraction gradually decreases and is practically nil after 14 days. The only formulation with attraction values above the control after 27 ageing days is Ataecina. This value demonstrates that the Ataecin attractant for flies continues even 4 weeks after being applied, and does not appear to cause repellence by containing insecticide in either case.

The Karate formulation shows no differences to the control after 21 and 27 days. This is likely because the applied formulation would have completely degraded.

Example 10. Field formulation efficacy test

A field test is performed to study the insecticidal activity of the 4% Ataecina formulation and the duration of its insecticidal effect.

The test is carried out on a variety of citrus varieties. The plot is divided into three subplots with 6x7 trees in an approximately square shape.

In all these subsections, one treatment type is performed. No chemical treatment takes place in the more easterly plot (the control subplot in FIG. 1). In the central plot (Karate subplot in FIG. 1), two standard treatments are performed on 20 September and 5 October with Karate Zeon 10% w/v and Nulure as the protein attractant. Karate is used at the 12.5 ml dose of this product in 10 litres of broth and Nulure at 150 ml per 10 litres of broth. The flow rate is about 100-150 ml per tree. A single treatment with 4% Ataecina at the 5% v/v dose is performed on 20 September in the third more westerly subplot. The code of the used Ataecina is:

## PS_NP * (10)_PMn (0.4) × 16

In the centre of each plot, a Teprhi Trap (Utilplas, Madrid, Spain) is primed with a Biolure female attractant (Suterra, Valencia), which is reviewed on a weekly basis.

Two damage counts are performed on fruit. The first on 11 October by inspecting fruit directly from the three central trees per plot. At the end of the test on 17 October, the fruit from the three trees marked in the centre of each plot are harvested. Fruit is taken to the laboratory to be checked and placed inside a chamber for 1 week at 22°C. After 1 week, the percentage of attacked fruit is determined and how many of them have developed *Ceratitis capitata* larvae.

Regarding the fly population caught in the follow-up traps, a single Ataecina application suffices to maintain fly populations at practically 0. In fact, no females are caught after its application until the test ends 27 days later. In the Karate-treated plot, the fly population reduces for 2 weeks, after which time it rises again. By repeating the Karate treatment, the fly population once again lowers. In the control plot, the fly population remains high throughout the test and surpasses the threshold of 1 fly per trap/day, which is considered a sufficient population level to cause considerable crop damage.

Table 1: Males and females (M/F) caught in the central traps in each plot with each treatment type.

|  | 20/09 | 27/09 | 4/10 | 11/10 | 17/10 |
|---|---|---|---|---|---|
| **Ataecina** | 0/1 | 0/0 | 1/0 | 1/0 | 0/0 |
| **Karate** | 1/1 | 0/0 | 1/0 | 2/1 | 1/0 |
| **Control** | 3/5 | 0/6 | 2/7 | 12/7 | 3/5 |

The reduction of the caught population in the control plot might be due to the proximity of the Karate-treated plot. In any case, we can see that Ataecina maintains the fly population at zero after 4 weeks of testing.

On fruit damage, the results of the two counts are shown in Table 2:

Table 2: Percentage of damaged fruit depending on the treatment performed during the 11 October count.

| Treatment | Fruits inspected on the tree | Damaged fruit % |
|---|---|---|
| **Ataecina** | 282 | 0,00 % |
| **Karate** | 799 | 0,25 % |
| **Control** | 240 | 0,83 % |

This first count is performed on all the fruit on the three trees in the centre of each plot. Fruits are only inspected visually because the phenological fruit status goes from the beginning of ripening, but they are still not ripe enough for harvesting.

The second fruit evaluation takes place on 17 October when full fruit maturity can be considered. The results appear in Table 3:

Table 3: Percentage of damaged fruit depending on the treatment performed on 17 October.

| Treatment | Inspected fruits | Fruit weight, (kg) | Damaged fruit, % | Fruits with larvae, % |
|---|---|---|---|---|
| **Ataecina** | 263 | 21,9 | 0,00% | 0,00% |
| **Karate** | 797 | 48,3 | 0,38% | 0,12% |
| **Control** | 342 | 25,2 | 3,21% | 2,34% |

Percentages followed by different letters in the same column significantly differ in the Chi square test with $P < 0.05$

As we can see, both the Karate and Ataecina treatments significantly reduce the damage caused by Mediterranean flies. However, this reduction is absolute for a single Ataecina treatment because no damaged fruit appear in either of the two evaluations made. The Karate treatment must be repeated 2 weeks after the first treatment because insecticidal activity on leaves disappears and captures increase. The percentage of damaged fruit that undergoes Karate treatment is acceptable for market levels, but is still higher than the fruit in the Ataecina-treated plots. It should also be noted that the single Ataecina treatment reduces fly populations at practically zero and avoids attacks by flies.

**Claims**

1. A nanodevice for the controlled release of molecules with insecticidal properties that comprises a protein-coated support, wherein said proteins comprise all the essential amino acids.

2. The nanodevice in claim 1 is **characterised by** having a diameter from 10 nm to 250 nm, 250 nm to 500 nm, 500 nm to 1 micron, but preferably from 25 nm to 150 nm.

3. The nanodevice any one of the preceding claims wherein the support is a mesoporous material, preferably mesoporous silica.

4. According to the above claims, the nanodevice support pore diameter goes from 0.5 nm to 10 nm, but preferably from 1.5 nm to 3.5 nm, and has a specific BET surface between 10 $m^2$/g and 1,500 $m^2$/g, but preferably from 200 $m^2$/g to 700 $m^2$/g.

5. The nanodevice according to any one of the preceding claims further comprising the substance to be released.

6. According to the previous claim, the substance to be released is selected from the list of synthetic insecticides, natural insecticides, or any mixtures thereof with ingestion activity.

7. According to the previous claims, the substance to be released is selected from the list of natural pyrethrum, the natural pyrethrins obtained from Chrysanthemum plans genus, and different synthetic pyrethroids, such as Allethrin, fenvalerate, fluvalinate, Cylfluthrin, Bifenthrin, Fenpropatine, Resveratrol, Sumithrin, Permethrin, Esfenvalerate, Cypermethrin, Lambda cyhalothin, Deltamethrin, Tefluthrin, Tetraprin and Tralomeine, and/or any of the mixtures thereof.

8. According to the previous claims, the ratio of the substance to be released goes from 0.01 to 0.50 g per support g.

9. The invention relates to a method to obtain the nanodevice (obtaining method) is in accordance with any of the above claims and comprises the steps of:

    a) synthesis of the silica mesoporous support
    b) suspension of the support in a solution of the substance to be released
    c) addition of excess protein to the above suspension

10. The obtaining method is in accordance with the above claim, where step (b) involves stirring for 1 to 48 h, but preferably for 10 to 30 h.

11. The obtaining method is in accordance with claims 9 and 10, where step (b) is carried out in a solvent selected from the list of water, methanol, ethanol, propanol, isopropanol, acetone and acetonitrile, and preferably water.

12. The obtaining method is in accordance with claims 7 to 11, where the functionalised protein/support ratio is 1-5 times in mass.

13. The obtaining method is in accordance with claims 8 to 12, where step (c) involves stirring for 0.5 to 20 h, but preferably for 1 to 10 h.

14. The obtaining method is in accordance with any of the above claims and comprises these steps:

    a) synthesis of the silica mesoporous support containing the substance to be released in a mini-emulsion stage.
    b) adding excess protein to the above suspension

15. The obtaining method, in accordance with the above claim, wherein step (b) is stirred for 1 to 48 hours, preferably 10 to 30 hours.

16. The obtaining method is in accordance with claim 14 where in step (b) is stirred for 1 to 48 hours, preferably for 10 to 30 hours.

17. Composition that comprises nanodevices is in accordance with claims 1 to 8.

18. Composition is in accordance with the previous claim, where this composition is insecticide.

19. Composition is in accordance with claims 17 to 18, and also comprises excipients for acceptable insecticides.

20. Composition is in accordance with claims 17 to 19, wherein said composition is in the form of a tablet, capsule, powder, granule, solution, suppository or syrup

21. The nanodevice is employed in accordance with claims 1 to 8 to manufacture an insecticide.

22. Its use is in accordance with the claims 1 to 22, where the insects to which it is applied are selected from the list of fruit flies (genera: *Ceratitis, Anastrepha, Bactocera, Dacus, Rhagoletis,* and other *tephritidae*, as well as the *Drosophilidae* family).


**Amended claims under Art. 19.1 PCT**

1. A nanodevice for the controlled release of molecules with insecticidal properties that comprises: a support of mesoporous material, preferably mesoporous silica; the insecticidal substance to be released; a protein coating that attracts certain insect families, such as tephritidae or drosophilidae.

2. The nanodevice in claim 1 is **characterised by** having a diameter from 10 nm to 250 nm, but preferably from 25 nm to 150 nm.

3. According to the above claims, the nanodevice support pore diameter goes from 0.5 nm to 10 nm, but preferably from 1.5 nm to 3.5 nm, and has a specific BET surface between 10 $m^2/g$ and 1,500 $m^2/g$, but preferably from 200 $m^2/g$ to 700 $m^2/g$.

4. According to the previous claim, the substance to be released is selected from the list of synthetic insecticides, natural insecticides, or any mixtures thereof with ingestion activity.

5. According to the previous claims, the substance to be released is selected from the list of natural pyrethrum, the natural pyrethrins obtained from Chrysanthemum plans genus, and different synthetic pyrethroids, such as Allethrin, fenvalerate, fluvalinate, Cylfluthrin, Bifenthrin, Fenpropatine, Resveratrol, Sumithrin, Permethrin, Esfenvalerate, Cypermethrin, Lambda cyhalothin, Deltamethrin, Tefluthrin, Tetraprin and Tralomeine, and/or any of the mixtures thereof.

6. According to the previous claims, the ratio of the substance to be released goes from 0.01 to 0.50 g per support g.

7. The invention relates to a method to obtain the nanodevice (obtaining method) is in accordance with any of the above claims and comprises the steps of:

    a) synthesis of the silica mesoporous support
    b) suspension of the support in a solution of the substance to be released
    c) addition of excess protein to the above suspension

8. The obtaining method is in accordance with the above claim, where step (b) involves stirring for 1 to 48 h, but preferably for 10 to 30 h.

9. The obtaining method is in accordance with claims 7 and 8, where step (b) is carried out in a solvent selected from the list of water, dichloromethane, methanol, ethanol, propanol, isopropanol, acetone and acetonitrile, and preferably water.

10. The obtaining method is in accordance with claims 7 to 9, where the functionalised protein/support ratio is 1-5 times in mass.

11. The obtaining method is in accordance with claims 7 to 10, where step (c) involves stirring for 0.5 to 20 h, but preferably for 1 to 10 h.

12. The obtaining method is in accordance with any of the above claims and comprises these steps:

   a) synthesis of the silica mesoporous support containing the substance to be released in a mini-emulsion stage using an oil phase containing pyrethroids or natural pyrethrum and an aqueous phase
   b) adding excess protein to the above suspension

13. The obtaining method, in accordance with the above claim, wherein step (a) involves a mini-emulsion containing a percentage of the oil phase in the water phase between 5% to 40%, but preferably from 20% to 40%.

14. The obtaining method is in accordance with claims 12 to 13, where the functionalised protein/support is 1-5 times in mass.

15. The obtaining method is in accordance with claims 12 to 13, where step (b) involves stirring for 0.5 to 20 h, but preferably 1 to 10 h.

16. Composition that comprises nanodevices is in accordance with claims 1 to 6.

17. Composition is in accordance with the previous claim, where this composition contains insecticide.

18. Composition is in accordance with claims 15 to 16, and also comprises excipients for acceptable insecticides.

19. The nanodevice is employed in accordance with claims 1 to 6 to manufacture an insecticide formulation.

20. Its use is in accordance with the above claims, where the insects to which it is applied are selected from the list of fruit flies (genera: *Ceratitis, Anastrepha, Bactocera, Dacus, Rhagoletis,* and other *tephritidae*, as well as the *Drosophilidae* family).

Figure 1.

Figure 2.

Figure 3.

Figure 4.

## Figure 5.

**% Mortality one hour after exposure**

| | 0d | 7 d | 14d | 21 d | 27d |
|---|---|---|---|---|---|
| Form 1 | 38% | 20% | 3% | 5% | 3% |
| Form 2 | 3% | 8% | 3% | 0% | 0% |
| Ataecina | 52% | 35% | 22% | 3% | 3% |
| Karate | 78% | 13% | 0% | 0% | 0% |
| Control | 3% | 3% | 0% | 0% | 0% |

## Figure 6.

**% Mortality three hours after exposure**

| | 0d | 7 d | 14d | 21 d | 27d |
|---|---|---|---|---|---|
| | | | 3horas | | |
| Form 1 | 68% | 33% | 3% | 18% | 0% |
| Form 2 | 19% | 13% | 8% | 0% | 0% |
| Ataecina | 84% | 45% | 36% | 15% | 3% |
| Karate | 93% | 38% | 0% | 0% | 0% |
| Control | 5% | 0% | 0% | 0% | 0% |

## Figure 7.

| | 0d | 7 d | 14d | 21 d | 27d |
|---|---|---|---|---|---|
| Form 1 | 65% | 40% | 3% | 13% | 0% |
| Form 2 | 24% | 13% | 10% | 0% | 0% |
| Ataecina | 84% | 28% | 20% | 13% | 5% |
| Karate | 95% | 30% | 5% | 0% | 0% |
| Control | 13% | 3% | 0% | 0% | 0% |

% Mortality 24 hour after exposure

## Figure 8.

% Atracción

## INTERNATIONAL SEARCH REPORT

| | International application No. |
|---|---|
| | PCT/ES2020/000051 |

**A. CLASSIFICATION OF SUBJECT MATTER**

**See extra sheet**

According to International Patent Classification (IPC) or to both national classification and IPC

**B. FIELDS SEARCHED**

Minimum documentation searched (classification system followed by classification symbols)
B01J, B82Y, A01N, A01P

Documentation searched other than minimum documentation to the extent that such documents are included in the fields searched

Electronic data base consulted during the international search (name of data base and, where practicable, search terms used)

EPODOC, INVENES, WPI, BIOSIS, MEDLINE, EMBASE, NPL, XPESP, PATENW

**C. DOCUMENTS CONSIDERED TO BE RELEVANT**

| Category* | Citation of document, with indication, where appropriate, of the relevant passages | Relevant to claim No. |
|---|---|---|
| X | US 2003108743 A1 (ANDERSON DAVID M) 12/06/2003; paragraphs [0019], [0051], [0300], [0339], [0355] - [0365]; claim 43. | 1, 2, 5-7 |
| A | US 2004076681 A1 (DENNIS DONN M *et al.*) 22/04/2004; paragraphs [0036] - [0047], [0054], [0074] - [0078]; examples; claims 15-18. | 1-22 |
| A | WO 2018096098 A1 (UNIV VALENCIA POLITECNICA *et al.*) 31/05/2018; page 2, line 9 - page 4, line 14; examples. | 1-22 |

☒ Further documents are listed in the continuation of Box C.       ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 12/01/2021 | **(13/01/2021)** |

| Name and mailing address of the ISA/ | Authorized officer |
|---|---|
| OFICINA ESPAÑOLA DE PATENTES Y MARCAS | N. Vera Gutierrez |
| Paseo de la Castellana, 75 - 28071 Madrid (España) | |
| Facsimile No.: 91 349 53 04 | Telephone No. 91 3495544 |

Form PCT/ISA/210 (second sheet) (January 2015)

**EP 4 066 933 A1**

## INTERNATIONAL SEARCH REPORT

International application No.

PCT/ES2020/000051

C (continuation). DOCUMENTS CONSIDERED TO BE RELEVANT

| Category * | Citation of documents, with indication, where appropriate, of the relevant passages | Relevant to claim No. |
|---|---|---|
| A | SCHLOSSBAUER A *et al.*, Biotin-avidin as a protease-responsive cap system for controlled guest release from colloidal mesoporous silica. Angewandte Chemie - International Edition 20090414 Wiley-vch Verlag Deu., 14/04/2009, Vol. 48, N° 17, pages 3092 - 3095, ISSN 1433-7851 (print), <DOI: 10.1002/anie.200805818> | 1-22 |
| A | US 2012021034 A1 (ZINK JEFFREY I *et al.*) 26/01/2012; paragraphs [0009], [0027]-[0065]. | 1-22 |
| A | ZHAO Y *et al.*, Mesoporous silica nanoparticle-based double drug delivery system for glucose-responsivecontrolled release of insulin and cyclic AMP. Journal of the American Chemical Society, American Chemical Society USA, 24/06/2009, Vol. 131, N° 24, pages 8398 - 8400, ISSN 0002-7863 (print), <DOI: 10.1021/ja901831u> | 1-22 |
| A | BERNARDOS A *et al.*, Enzyme-responsive controlled release using mesoporous silica supports capped with lactose. Angewandte Chemie – International Edition 20090727 Wiley-vch Verlag Deu., 27/07/2009, Vol. 48, N° 32, pages 5884 - 5887, ISSN 1433-7851 (print), <DOI: 10.1002/anie.200900880> | 1-22 |

Form PCT/ISA/210 (continuation of second sheet) (January 2015)

18

| INTERNATIONAL SEARCH REPORT | | International application No. | |
|---|---|---|---|
| Information on patent family members | | PCT/ES2020/000051 | |
| Patent document cited in the search report | Publication date | Patent family member(s) | Publication date |
| US2003108743 A1 | 12.06.2003 | AU2009201314 A1 | 23.04.2009 |
| | | AU2009201314B B2 | 28.04.2011 |
| | | US2004201117 A1 | 14.10.2004 |
| | | US6989195 B2 | 24.01.2006 |
| | | US6482517 B1 | 19.11.2002 |
| | | US6638621 B2 | 28.10.2003 |
| | | US2006073333 A1 | 06.04.2006 |
| | | US7105229 B2 | 12.09.2006 |
| | | WO03106168 A1 | 24.12.2003 |
| | | JP2005529949 A | 06.10.2005 |
| | | EP1534512 A1 | 01.06.2005 |
| | | EP1534512 A4 | 29.04.2009 |
| | | CA2488643 A1 | 24.12.2003 |
| | | AU2002312476 A1 | 31.12.2003 |
| | | AU2002312476B B2 | 23.04.2009 |
| US2004076681 A1 | 22.04.2004 | WO2005098429 A2 | 20.10.2005 |
| | | WO2005098429 A3 | 26.05.2006 |
| | | US2005037374 A1 | 17.02.2005 |
| | | US2006040318 A1 | 23.02.2006 |
| | | US7052854 B2 | 30.05.2006 |
| | | US2006160134 A1 | 20.07.2006 |
| | | US7195780 B2 | 27.03.2007 |
| | | WO2004037316 A2 | 06.05.2004 |
| | | WO2004037316 A3 | 07.10.2004 |
| | | AU2003290524 A1 | 13.05.2004 |
| | | AU2003290524 A8 | 13.05.2004 |
| | | EP1718971 A2 | 08.11.2006 |
| WO2018096098 A1 | 31.05.2018 | CZ2016735 A3 | 06.06.2018 |
| | | EP3544594 A1 | 02.10.2019 |
| US2012021034 A1 | 26.01.2012 | WO2010071831 A2 | 24.06.2010 |
| | | WO2010071831 A3 | 21.10.2010 |

Form PCT/ISA/210 (patent family annex) (January 2015)

## INTERNATIONAL SEARCH REPORT

| International application No. |
| --- |
| PCT/ES2020/000051 |

### CLASSIFICATION OF SUBJECT MATTER

*B01J13/20* (2006.01)
*B82Y5/00* (2011.01)
*A01N25/08* (2006.01)
*A01N25/28* (2006.01)
*A01N53/12* (2006.01)
*A01P7/04* (2006.01)

Form PCT/ISA/210 (extra sheet) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- ES 2237668 T3
- WO 2017091096 A2
- EP 2662069 A2
- WO 2018096098 A

## Non-patent literature cited in the description

- Ceratitis capitata is the most serious pest for citrus and many other fruit in the majority of countries with a warm, Mediterranean tropical or subtropical climate. *EPPO Bulletin [0250-8052,* 2011, vol. 41 (3), 340
- **PRIMO MILLO, EDUARDO.** *Agriculture,* 2004, (867), 790-703
- *Plan Valenciano de Actuacion Contra la Mosca de la Fruta (Ceratitis capitata Weidemann),* 2004
- **COUSO FERRER ; F. AROURI RABAH ; BEROIZ, B ; PERERA, N ; CERVERA, A ; NAVARRO-LLOPIS, V ; CATAÑERA, P ; HERNANDEZ-CRESPO, C ; ORTEGA F.** Cross-Resistance to insecticides in a Malathion-Resistant Strain of Ceratitis Capitata (diptera: Tephritidae). *Journal of Economic Entomology.,* 2011, vol. 104 (4), 1349-1358
- Manejo Integrado de Plagas. **ROMERO F.** Las bases, los conceptos y su mercantilizacion. Universidad Autónoma de Chapingo, 2004, 1-37
- **WANDHWA P ; RANST EV ; DAMME PV.** Pyrethrum (Chrysanhemum cinerariaefolium Vis.) Cultivation in West Kenya: origin, ecology conditions and management. *Industrial Crops and Products,* 1996, vol. 5, 307-322
- **HILL IR ; LEAHEY J P.** The pyrethroid insecticides. Taylor & Francis, 1985, 151-247
- **WEHLING et al.** A critical study: assessment of the effect of silica particles from 15 to 500 nm on bacterial viability. *Environmental Pollution,* 2013, vol. 176, 292-299
- **LIU et al.** Porous hollow silica nanoparticles as a controlled delivery system for water-soluble pesticide. *Materials Research Bulletin,* 2006, vol. 41, 2268-2275
- **CHEN et al.** Slow-Release Formulation of a New Biological Pesticide, Pyoluteorin, With Mesoporous Silica. *J Agric. Food Chem.,* 2011, vol. 59 (1), 307-311
- **WANG et al.** Construction and evaluation of a controlled release delivery system of Abamectin using porous silica nanoparticles as carriers. *Nanometric Research Letters,* December 2014, vol. 9, 655